(19) Europäisches Patentamt
European Patent Office
Office européen des brevets

(11) Veröffentlichungsnummer: **0 009 232**
**B1**

# (12) EUROPÄISCHE PATENTSCHRIFT

(45) Veröffentlichungstag der Patentschrift: **06.05.81**

(21) Anmeldenummer: **79103501.7**

(22) Anmeldetag: **18.09.79**

(51) Int. Cl.[3]: **C 07 J 19/00,**
C 07 J 53/00,
A 61 K 31/705

(54) **4,5-Methano-bufadienolid-rhamnoside, Verfahren zu deren Herstellung und diese enthaltende Arzneimittel.**

(30) Priorität: **21.09.78 DE 2841074**

(43) Veröffentlichungstag der Anmeldung:
**02.04.80 Patentblatt 80/7**

(45) Bekanntmachung des Hinweises auf die Patenterteilung:
**06.05.81 Patentblatt 81/18**

(84) Benannte Vertragsstaaten:
**AT BE CH DE FR GB IT LU NL**

(56) Entgegenhaltungen:
**Keine**

(73) Patentinhaber: **BASF Aktiengesellschaft**
**Carl-Bosch-Strasse 38**
**D-6700 Ludwigshafen (DE)**

(72) Erfinder: **Albrecht, Hans Peter, Dr.**
**Brombeerweg 5**
**D-6940 Weinheim (DE)**
Erfinder: **von Philipsborn, Gerda, Dr.**
**Naechstenbacher Weg 35**
**D-6940 Weinheim (DE)**
Erfinder: **Siebeneick, Hans Ulrich, Dr.**
**von Kiefferstrasse 92**
**D-6700 Ludwigshafen (DE)**
Erfinder: **Raschack, Manfred, Dr.**
**Donnersbergstrasse 7**
**D-6714 Weisenheim a. Sand (DE)**

**EP 0 009 232 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 4,5-Methano-bufadienolid-rhamnoside, Verfahren zu deren Herstellung und diese enthaltende Arzneimittel

Steroide, die an zwei nachbarständigen Kohlenstoffatomen eine Methylenbrücke tragen, sind bereits bekannt. Eine solche Verbindung ist das Cyproteronacetat, welches als Antiandrogen verwendet wird. Herzwirksame Steroide mit Methylenbrücken sind bislang nicht beschrieben worden.

Gegenstand der Erfindung sind neue 4,5-Methano-bufandienolidrhamnoside der Formel I

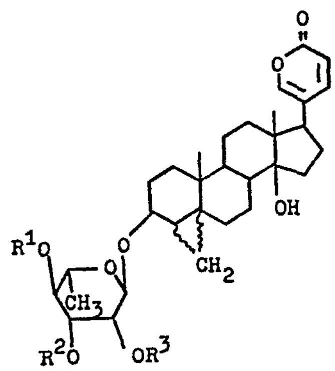

I,

worin $R^1$ ein Wasserstoffatom oder einen Alkyl- oder Acylrest mit jeweils 1 bis Kohlenstoffatomen bedeutet, $R^2$ und $R^3$ gleich oder verschieden sind und Wasserstoffatome, Alkyl- oder Acylrest mit jeweils 1 bis 4 Kohlenstoffatomen oder beide zusammen den Rest

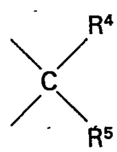

darstellen, worin $R^4$ und $R^5$ gleich oder verschieden sind und Wasserstoffatome oder Alkyl- oder Alkoxygruppen mit jeweils 1 bis 2 Kohlenstoffatomen darstellen.

Gegenstand der Erfindung ist weiter ein Verfahren zur Herstellung der Verbindungen der Formel I, welches dadurch gekennzeichnet ist, daß man

a) ein 3,14-Dihydroxy-4,5-methano-14$\beta$-bufa-20,22-dienolid der allgemeinen Formel II

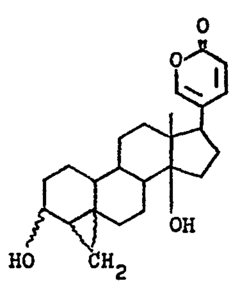

II

mit einem Rhamnosederivat der allgemeinen Formel III

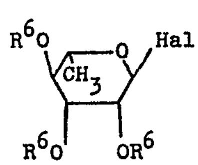

III,

worin $R^6$ Acetylgruppen oder Benzoylgruppen darstellen und Hal ein Halogenatom bedeutet, umsetzt und anschließend gegebenenfalls die so erhaltenen Verbindungen in an sich bekannter Weise verseift und danach verethert und/oder in sie den Rest

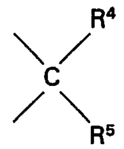

einführt, worin $R^4$ und $R^5$ dasselbe wie oben bedeuten, oder

b) — falls die Verbindung der allgemeinen Formel I ein cis-Methano-proscillaridinderivat darstellt — an eine Verbindung der allgemeinen Formel IV

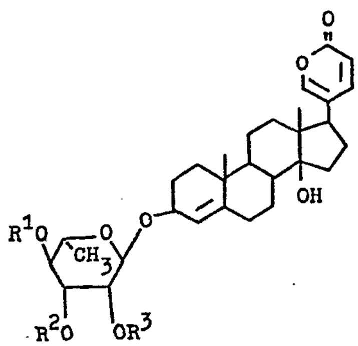

IV

worin $R^1$, $R^2$ und $R^3$ dasselbe wie oben bedeuten, Methylen addiert.

Gegenstand der Erfindung sind weiter Arzneimittel, welche eine Verbindung der Formel I enthalten.

In den Verbindungen I können $R^1$ bis $R^3$ außer Wasserstoffatomen, Methyl-, Ethyl-, n-Propyl-, iso-Propyl-, n-Butyl-, iso-Butyl, Formyl-, Acetyl-, Propionyl-, n-Butyryl- oder Isobutyrylreste darstellen. $R^4$ und $R^5$ können Formyl-, Acetyl, Methoxy-, Ethoxy-, Methyl- oder Ethylreste sein.

Die steroidalen Aglykone II werden zweckmäßig mit einem Überschuß (vorzugsweise 3 bis 5 Mol) an acylierter 1-Halogenrhamnose III umgesetzt. Die Reaktion gelingt gut bei 0 bis 30°C, so daß sie bei Raumtemperatur durchgeführt werden kann. Geeignete inerte Lösungsmittel für die Reaktion sind Methylenchlorid, Ether, Tetrahydrofuran, Acetonitril, Nitromethan und vorzugsweise Dichlorethan. Die Reaktionsdauer ist temperaturabhängig und beträgt in der Regel 3 bis 24 Stunden. Zweckmäßigerweise wird die Umsetzung in Gegenwart von Säureakzeptoren durchgeführt, wie Cyaniden, Oxiden und Carbonaten von Metallen der 1. und 2. Nebengruppe des Periodensystems. Beispiele dafür sind Silber(I)oxid, Siber(I)carbonat, Quecksilber(II)oxid, Quecksilber(II)carbonat, Cadmiumcarbonat und vorzugsweise Quecksilber(II)cyanid.

Die so erhaltenen Verbindungen können in an sich bekannter Weise, vorzugsweise mit methanolischem Ammoniak bei Raumtemperatur oder mit Kaliumhydrogencarbonat in wäßrigem Methanol bei 20 bis 70°C entacyliert werden.

Auch die Veretherung der freien OH-Gruppen und die Einführung des Restes

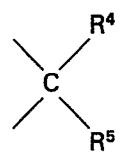

erfolgt in an sich bekannter Weise.

Die Methylenaddition an die Verbindungen IV erfolgt zweckmäßig durch Umsetzen der Verbindungen IV mit Jodmethyl-zinkiodid nach Simmons-Smith (vgl. H.E. Simmons et at. Organic Reactions *20*, 1, John Wiley and Sons, 1973). In der Praxis wird vorzugsweise ein durch Umsetzen von Zinkstaub und Kupfersalz, insbesondere CuCl, in Ether bei Rückflußtemperatur erhaltenes Zink-Kupfer-Paar (vgl. R.J. Rawson, I.T. Harrison, J. Org. Chem. *35*, 2057 (1970)) in einer Stickstoffatmosphäre nacheinander mit Diiodmethan und einer Lösung des Proscillaridinderivates in einem inerten Lösungsmittel, vorzugsweise Tetrahydrofuran, versetzt. Das Zink-Kupfer-Paar und das Diiodmethan werden in einem etwa 2- bis 30-, vorzugsweise 3- bis 10-fachen Überschuß verwendet. Die Reaktion läuft gut bei 30 bis 65°C und ist in der Regel nach 1 bis 24 Stunden beendet.

Die Methylenaddition erfolgt stereospezifisch an der 4,5-Doppelbindung des Proscillaridinderivates in cis-Stellung zur 3-0-Funktion. Es ist sehr überraschend, daß der ungesättigte Lactonring dabei nicht angegriffen wird.

Die neuen Verbindungen steigern die Kontraktionskraft des Herzens und sind für die Therapie der Herzinsuffizienz geeignet.

Im Vergleich zu den bisher in die Therapie eingeführten Herzglykosiden besitzen sie eine größere

Wirkstärke und eine erhöhte therapeutische Breite.

Die Wirkstärke der erfindungsgemäßen Verbindungen, d.h. die Konktraktionskraftzunahme am elektrisch gereizten und spontanschlagenden Vorhof des Meerschweinchens ist 4- bis 50-mal größer als bei bisher therapeutisch verwendeten Herzglykosiden.

Der Quotient aus arrhythmogen wirksamer Dosis und der für die Kontraktionskraftzunahme am spontanschlagenden Vorhof benötigten Dosis, ist 2- bis 10-mal höher als bei in die Therapie eingeführten Herzglykosiden. Weiter zeichnen sie sich durch eine große Säurestabilität aus.

Die erfindungsgemäßen Verbindungen können in üblicher Weise oral oder intravenös verabfolgt werden.

Die Dosierung hängt vom Alter, Zustand und Gewicht des Patienten sowie von der Applikationsart ab. In der Regel beträgt die tägliche Wirkstoffdosis zwischen etwa 0,003 und 0,06 mg/kg Körpergewicht sowohl bei oraler als auch intravenöser Gabe.

Die neuen Verbindungen können in den gebräuchlichen galenischen Applikationsformen fest oder flüssig angewendet werden, z.B. als Tabletten, Kapseln, Pulver, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren und/oder Lösungsmitteln verarbeitet werden (vgl. L.G. Goodman, A. Gilman: The Pharmacological Basis of Therapeutics).

Die Verbindungen der allgemeinen Formel II sind noch nicht beschrieben worden. Sie lassen sich durch Methylen-Addition an Verbindungen der allgemeinen Formel

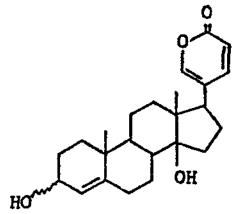

in der hier beschriebenen Weise herstellen. Dabei entstehen jedoch nur die 3α-Hydroxy-4α,5-methano- und die 3β-Hydroxy-4β,5-methano-Verbindungen. Aus diesen erhält man jedoch durch Oxidation mit Chromsäure/Schwefelsäure in Aceton nach Jones (J. Chem. Soc. *1953*, 2555) und anschließende Reduktion mit Natriumborhydrid in Methanol bei Raumtemperatur die 3α-Hydroxy-4β,5-methano- und die 3β-Hydroxy-4α,5-methano-Verbindungen.

Die nachstehenden Beispiele erläutern die Erfindung. Die Rf-Werte wurden an Kieselgel-Fertigplatten der Fa. Merck, Darmstadt, mit Methylenchlorid/Aceton = 5:1 (Fließmittel A) und Methylenchlorid/Methanol = 10:1 (Fließmittel B) bestimmt.

### Beispiel 1

a) 0,8 g (2,0 mMol) 3β,14-Dihydroxy-4β,5-methano-5β,14β-bufa-20,22-dienolid (Fp = 172 bis 180°C) in 40 ml wasserfreiem 1,2-Dichlorethan werden mit 4,0 g (7,5 mMol) 2,3,4-Tri-O-benzoyl-α-L-rhamnopyranosylbromid, 4,0 g Quecksilber(II)cyanid und 5 g Molekularsieb A 4 versetzt. Das Reaktionsgemisch wird bei Raumtemperatur gerührt und der Verlauf der Reaktion mittels DC (Fließmittel A) verfolgt.

Nach 1,5 Stunden wird mit Chloroform (600 ml) versetzt, über Kieselgur filtriert und das Filtrat mit 20%iger wäßriger Kaliumiodid-Lösung (2 x 150 ml) und Wasser (2 x 100 ml) gewaschen, über Natriumsulfat getrocknet und eingedampft.

Chromatographie des Rückstands an einer Kieselgelsäule (Elution mit Methylenchloride/Aceton = 10:1) gab 14 - Hydroxy - 4β,5 - methano - 3β[(2,3,4 - tri - O - benzoyl - α - L-rhamnopyranosyl)oxy] - 5β,14β - bufa - 20,22 - dienolid.

Schmelzpunkt: 153—158°C (Methylenchlorid/Ether/Hexan),

$[\alpha]_D^{20} = +18°$ (c = 0,5 in Chloroform),

UV (Methanol): $\lambda_{max}$ (lgε) = 288 (4,21) 283 (3,82)

298 (3,65)

Rf = 0,56 (Fließmittel A).

b) 857 mg (1 mMol) der gemäß a) erhaltenen Verbindung in 50 ml Methanol werden mit 5 ml einer 5%igen wäßrigen Kaliumhydrogencarbonat-Lösung versetzt. Nach 70 Stunden bei Raumtemperatur wird mit 300 ml Chloroform versetzt, mit wenig Wasser gewaschen und eingedampft.

Chromatographie des Rückstands an einer Kieselgelsäule (Elution mit Methylenchlorid/Methanol 10:1) und Kristallisation des Rückstandes aus Ethanol/Wasser gibt 305 mg (56%) 14-Hydroxy-4β,5-methano-3β-[(α-L-rhamnopyranosyl)oxy]-5β,14β-bufa-20,22-dienolid.

Schmelzpunkt: 251—258°C,

$[\alpha]_D^{20} = -105°$ (c = 0,5 in Chloroform/Methanol 10:1),

UV (Methanol): $\lambda_{max}$ (lg$\varepsilon$) = 297 (3,75)
Rf = 0,17 (Fließmittel B).
$C_{21}H_{44}O_8$ (544,7) Ber.: C 68,36 H 8,14
Gef.: C 67,8 H 8,2

Beispiel 2

In einer Stickstoffatmosphäre wird eine Suspension von 6,5 g (100 gAtom) Zinkstaub und 1,0 g (10 mMol) Kupfer(I)chlorid in 30 ml Ether unter kräftigem Rühren bei einer Badtemperatur von 40 bis 45°C auf Rückflußtemperatur erwärmt. Nach 30 Minuten werden 8,1 ml (100 mMol) Diiodmethan im Verlauf von 5 bis 10 Minuten zugetropft, anschließend wird eine Lösung von 5,7 g (10 mMol) 14-Hydroxy - 3$\beta$ - [(2,3,0 - isopropyliden - $\alpha$ - L - rhamnopyranosyl)oxy] - 14$\beta$ - bufa - 4,20,22 - trienolid (= Proscillaridin-2',3'-acetonid) in 50 ml Tetrahydrofuran langsam zugegeben.

Die Reaktion wird mittels DC (Fließmittel A) verfolgt. Nach 3 bis 4 Stunden bei einer Badtemperatur von 45—55°C ist die Umsetzung beendet. Das Reaktionsgemisch wird mit Tetrahydrofuran auf ein Volumen von 100 ml gebracht und unter Eiskühlung langsam mit 10 ml einer gesättigten wäßrigen Ammoniumchlorid-Lösung versetzt und über Kieselgur filtriert. Das Filtrat wird in Methylenchlorid (100 ml) aufgenommen und mit 10%iger wäßriger Natriumthiosulfat-Lösung (2 x 100 ml), gesättigter wäßriger Natriumhydrogencarbonat-Lösung (100 ml) und Wasser (2 x 100 ml) gewaschen, über Natriumsulfat getrocknet und im Vakuum eingedampft. Chromatographie des Rückstands an einer Kieselgesäule mit Methylendichlorid/Aceton = 8:1 und Kristallisation der Reinfraktion aus Ethanol ergibt 2,85 g (49%) 14-Hydroxy-4$\beta$,5-methano-3$\beta$-[(2,3-0-isopropyliden-$\alpha$-L-rhamnopyranosyl)oxy]-5$\beta$,14$\beta$-bufa-20,22-dienolid.

Schmelzpunkt: 229—232°C,
$[\alpha]_D^{20}$ = −94° (c = 0,5 in Chloroform)
UV (Methanol): $\lambda_{max}$ (lg$\varepsilon$) = 298 nm (3,75),
Rf = 0,54 (Fließmittel B).
$C_{34}H_{48}O_8$ (584,7) Ber.: C 69,84 H 8,27
Gef.: C 69,57 H 8,11

Analog erhält man:

14 - Hydroxy - 4$\beta$,5 - methano - 3$\beta$ - [(2,3 - 0 - isopropyliden - 4 - 0 - methyl - $\alpha$ - L - rhamnopyranosyl)oxy] - 5$\beta$,14$\beta$ - bufa - 20,22 - dienolid, Ausbeute 46%.

Schmelzpunkt; 160—163°C (Methylenchlorid/Ether/Hexan),
$[\alpha]_D^{20}$ = −96° (c = 0,5 in Chloroform),
UV (Methanol): $\lambda_{max}$ (lg$\varepsilon$) = 298 nm (3,75),
Rf = 0,47 (Fließmittel A)
$C_{35}H_{50}O_8$ (598,8) Ber.: C 70,20 H 8,42
Gef.: C 70,3 H 8,5

14 - Hydroxy - 4$\beta$,5 - methano - 3$\beta$ - [(2,3 - 0 - ethoxymethyliden - $\alpha$ - L - rhamnopyranosyl) oxy] - 5$\beta$,14$\beta$ - bufa - 20,22 - dienolid, Ausbeute 41%.

Schmelzpunkt: 245—250°C (Methylenchlorid/Methanol/Ether),
$[\alpha]_D^{20}$ = −80° (c = 0,5 in Chloroform),
UV (Methanol): $\lambda_{max}$ (lg$\varepsilon$) = 2,98 nm (3,76)
Rf = 0,43 (Fließmittel B),
$C_{34}H_{48}O_9$ (600,7) Ber.: C 67,97 H 8,05
$C_{34}H_{48}O_9$ (600,7) Gef.: C 67,75 H 8,00

Beispiel 3

2,9 g (5.0 mMol) 14 - Hydroxy - 4$\beta$,5 - methano - 3$\beta$ - [(2,3 - 0 - isopropyliden - $\alpha$ - L-rhamnopyranosyl)oxy]-5$\beta$,14$\beta$-bufa-20,22-dienolid (Beispiel 2) in 100 ml Tetrahydrofuran werden unter Rühren bei Raumtemperatur mit 20 ml 1N HCl versetzt. Der Verlauf der Reaktion wird mittels DC (Fließmittel B) verfolgt. Nach 32 Stunden wird durch Zugabe von 2 g Natriumhydrogencarbonat neutralisiert. Das Reaktionsgemisch wird in 800 ml Chloroform/Methanol (10:1) aufgenommen, mit wenig Wasser gewaschen und im Vakuum eingedampft. Chromatographie des Rückstand an einer Kieselgelsäule (Elution mit Methylendichlorid/Methanol = 10:1) und anschließende Kristallisation der Reinfraktion aus Ethanol gibt 1,4 g (51%) 14-Hydroxy-4$\beta$,5-methano-3$\beta$](α-L-rhamnopyranosyl)oxy]-5$\beta$,14$\beta$-bufa-20,22-dienolid.

Schmelzpunkt: 249-253°C,
$[\alpha]_D^{20}$ = −114° (c = 0,5 in Methanol/Chloroform = 10:1),
UV (Methanol): $\lambda_{max}$ (lg$\varepsilon$) = 297 (3,75),
Rf = 0,17 (Fließmittel B).

Analog erhält man:

14 - Hydroxy - 4$\beta$,5 - methano - 3$\beta$ - [(4 - 0 - methyl - $\alpha$ - L - rhamnopyranosyl)oxy] - 5$\beta$,14$\beta$-bufa - 20,22 - dienolid, Ausbeute 41%.

Schmelzpunkt: 220—234°C,
$[\alpha]_D^{20}$ = −109° (c = 0,5 in Chloroform),

UV (Methanol): $\lambda_{max}$ (lg$\varepsilon$) = 298 nm (3,76),
Rf = 0,28 (Fließmittel B)
$C_{32}H_{46}O_6$ (558,7) Ber.: C 68,79 H 8,30
Gef.: C 68,8 H,83

Beispiel 4

Auf einer Tablettenpresse werden in üblicher Weise Tabletten folgender Zusammensetzung gepreßt:

0,20 mg 14 - Hydroxy - 4$\beta$,5 - methano - 3$\beta$ - [($\alpha$ - L - rhamnopyranosyl)oxy] - 5$\beta$,14$\beta$-bufa - 20,22 - dienolid
15,0 mg Maisstärke
1,35 mg Gelatine
4,5 mg Milchzucker
2,25 mg Talk
0,22 mg Aerosil® (chemisch reine Kieselsäure in submikroskopisch feiner Verteilung)
0,67 mg Kartoffelstärke (als 6%iger Kleister).

Beispiel 5

In üblicher Weise werden Dragees folgender Zusammensetzung hergestellt:

0,30 mg 14 - Hydroxy - 4$\beta$,5 - methano - 3$\beta$ - [(2,3 - O - isopropyliden - $\alpha$ - L - rhamnopyranosyl)oxy] - 5$\beta$,14$\beta$,bufa - 20,22 - dienolid
17,0 mg Kernmasse
16,0 mg Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. *1962*, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 6

1 g 14 - Hydroxy - 4$\beta$,5 - methano - 3$\beta$ - [(4 - O - methyl - $\alpha$ - L - rhamnopyranosyl) - oxy] - 5$\beta$,14$\beta$-bufa 20,22-dienolid werden in 10 l Wasser gelöst. Die Lösung wird mit 0,1 N Natriumacetat auf pH 3,5 eingestellt und mit Kochsalz isotonisch eingestellt. Danach wird sie in 2 ml fassende Ampullen steril abgefüllt.

**Patentansprüche für die Vertragsstaaten: BE, CH, DE, FR, GB, IT, LU, NL.**

1. 4,5-Methano-bufadienolid-rhamnoside der Formel I

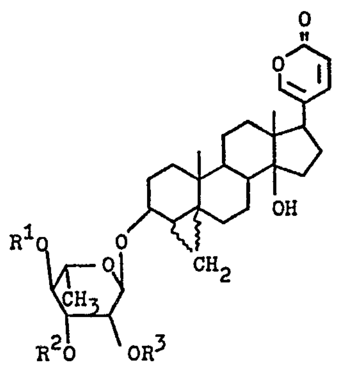

I,

worin $R^1$ ein Wasserstoffatom oder einen Alkyl- oder Acylrest mit jeweils 1 bis 4 Kohlenstoffatomen bedeutet,

$R^2$ und $R^3$ gleich oder verschieden sind und Wasserstoffatome, Alkyl- oder Acylreste mit jeweils 1 bis 4 Kohlenstoffatomen oder beide zusammen den Rest

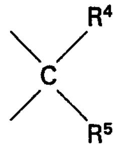

darstellen, worin $R^4$ und $R^5$ gleich oder verschieden sind und Wasserstoffatome oder Alkyl- oder Alkoxy-

gruppen mit jeweils 1 bis 2 Kohlenstoffatomen darstellen.

2. Verfahren zur Herstellung der Verbindungen der Formel I gemäß Anspruch 1, dadurch gekennzeichnet, daß man

a) ein 3,14-Dihydroxy-4,5-methano-14$\beta$-bufa-20,22-dienolid der allgemeinen Formel II

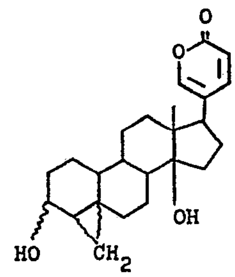

II

mit einem Rhamnosederivat der allgemeinen Formel III

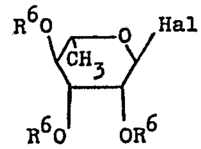

III

worin $R^6$ Acetylgruppen oder Benzoylgruppen darstellen und Hal ein Halogenatom bedeutet, umsetzt und anschließend gegebenenfalls die so erhaltenen Verbindungen in an sich bekannter Weise verseift und danach verethert und/oder in sie den Rest

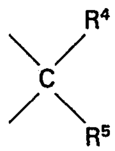

einführt, worin $R^4$ und $R^5$ daselbe wie oben bedeuten, oder

b) — falls die Verbindung der allgemeinen Formel I ein cis-Methano-proscillaridinderivat darstellt — an eine Verbindung der allgemeinen Formel IV

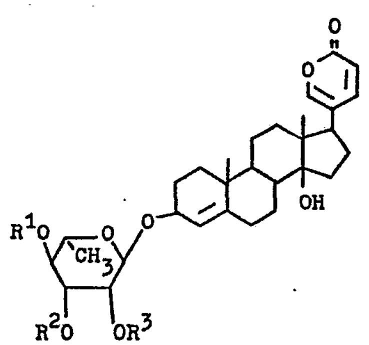

IV

worin $R^1$, $R^2$ und $R^3$ dasselbe wie oben bedeuten, Methylen addiert.

3. Arzneimittel, enthaltend eine Verbindung gemäß Anspruch 1.

4. Die Verbindungen gemäß Anspruch 1 zur Verwendung bei der Bekämpfung von Herzinsuffizienz.

5. 14-Hydroxy-4$\beta$,5-methano-3$\beta$-[($\alpha$-L-rhamnopyranosyl)oxy]-5$\beta$,14$\beta$-bufa-20,22-dienolid.

6. 14-Hydroxy-4$\beta$,5-methano-3$\beta$-[(2,3-0-isopropyliden-$\alpha$-L-rhamnopyranosyl)oxy]-5$\beta$,14$\beta$-bufa-20,22-dienolid.

7. 14-Hydroxy-4$\beta$,5-methano-3$\beta$-[(2,3-0-isopropyliden-4-0-methyl-$\alpha$-L-rhamnopyranosyl)oxy]-5$\beta$,14$\beta$-bufa-20,22-dienolid.

8. 14-Hydroxy-4$\beta$,5-methano-3$\beta$-[(2,3-0-ethoxymethyliden-$\alpha$-L-rhamnopyranosyl)oxy]-5$\beta$,14$\beta$-bufa-20,22-dienolid.

9. 14-Hydroxy-4$\beta$,5-methano-3$\beta$-[(4-0-methyl-$\alpha$-L-rhamnopyranosyl)oxy]-5$\beta$,14$\beta$-bufa-20,22-dienolid.

**Revendications pour les Etats contractants: BE, CH, DE, FR, GB, IT, LU, NL.**

1. 4,5-méthano-bufadienolide-rhamnoside de formule I

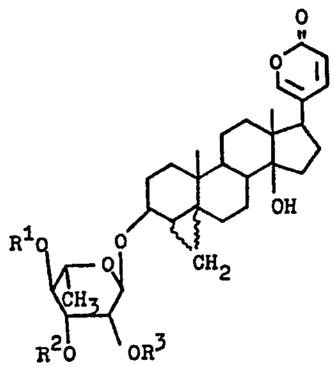

dans laquelle $R^1$ représente un atome d'hydrogène ou un reste alkyle ou acyle ayant 1 à 4 atomes de carbone, $R^2$ et $R^3$ sont identiques ou différents et représentent des atomes d'hydrogène, des restes alkyle ou acyle ayant 1 à 4 atomes de carbone ou bien les deux ensemble, le reste

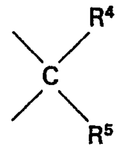

où $R^4$ et $R^5$ sont identiques ou différents et représentent des atomes d'hydrogène ou des groupes alkyle ou alkoxy ayant 1 à 2 atomes de carbone.

2. Procédé de préparation des composés de formule I selon la revendication 1, caractérisé par le fait que

a) on fait réagir un 3,14-dihydroxy-4,5-méthano-14β-bufa-20,22-diénolide de formule générale II

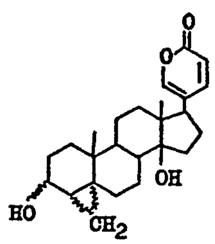

avec un dérivé de rhamnoside de formule générale III

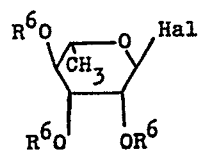

dans laquelle $R^6$ représente des groupes acétyle ou benzoyle et Hal un atome d'halogène puis, éventuellement, on saponifie de manière connue les composés ainsi obtenus et enfin on estérifie et/ou on y introduit le reste

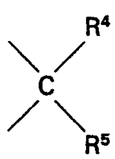,

dans lequel $R^4$ et $R^5$ ont la même signification que ci-dessus, ou

b) dans le cas où le composé de formule générale I représente un dérivé de cis-méthano-

proscillaridine — on additionne de méthylène un composé de formule générale IV

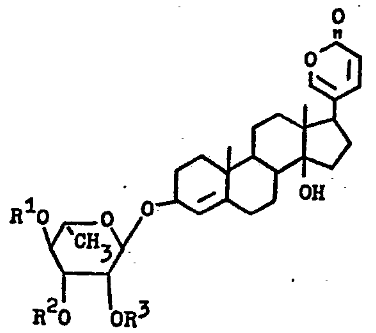

dans laquelle $R^1$, $R^2$ et $R^3$ ont la même signification que ci-dessus.

3. Médicament contenant un composé selon la revendication 1.

4. Les composés selon la revendication 1 pour utilisation lors de la lutte contre l'insuffisance cardiaque.

5. 14-hydroxy-4β,5-methano-3β[(α-L-rhamnopyranosyl)oxy]-5β,14β-bufa-20,22-diénolide.

6. 14-hydroxy-4β,5-methano-3β-[(2,3-0-isopropyliden-α-L-rhamnopyranosyl)oxy]-5β,14β-bufa-20,22-diénolide.

7. 14-hydroxy-4β,5-méthano-3β-[(2,3-0-isopropyliden-4-0-méthyl-α-L-rhamnopyransoyl)oxy]-5β,14β-bufa-20,22-diénolide.

8. 14-hydroxy-4β,5-méthano-3β-[(2,3-0-éthoxyméthyliden-α-L-rhamnopyranosyl)oxy]-5β,14β-bufa-20,22-diénolide.

9. 14-hydroxy-4β,5-méthano-3β-[(4-0-méthyl-α-L-rhamnopyranosyl)oxy]-15β,14β-bufa-20,22-diénolide.

**Claims for the Contracting states: BE, CH, DE, FR, GB, IT, LU, NL**

1. A 4,5-Methano-bufadienolide-rhamnoside of the formula I

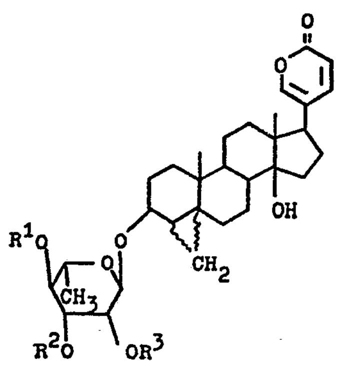

I

where $R^1$ is hydrogen or alkyl of 1 to 4 carbon atoms or acyl of 1 to 4 carbon atoms, and $R^2$ and $R^3$ are identical or different and each is hydrogen or alkyl of 1 to 4 carbon atoms or acyl of 1 to 4 carbon atoms, or both together are

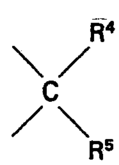

where $R^4$ and $R^5$ are identical or different and each is hydrogen or alkyl or alkoxy of 1 or 2 carbon atoms.

2. A process for the preparation of a compound of the formula I as claimed in claim 1, wherein

a) 1 3,14-dihydroxy-4,5-methano-14β-bufa-20,22-dienolide of the general formula II

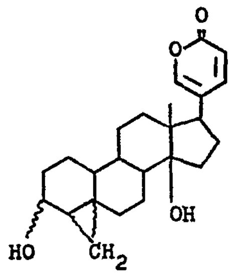

II

is reacted with a rhamnose derivative of the general formula III

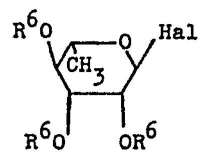

III

where the $R^6$ groups are acetyl or benzoyl and Hal is halogen, and if desired the resulting compound is hydrolyzed in a conventional manner and then if desired etherified and/or if desired a radical

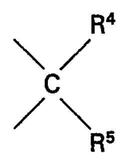

where $R^4$ and $R^5$ have the above meanings, is introduced into the resulting compound, or

b) if the compound of the general formula I to be produced is a cis-methano-proscillaridine derivative, a methylene group is introduced into a compound of the general formula IV

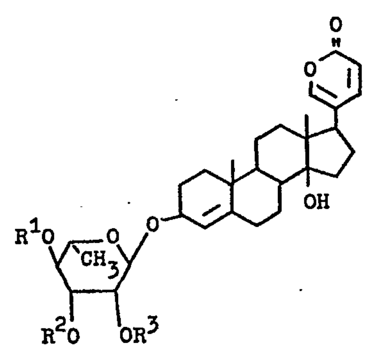

IV

where $R^1$, $R^2$ and $R^3$ have the same meanings as above.

3. A therapeutic composition containing a compound according to claim 1.

4. The compounds according to claim 1 for use in the treatment of cardiac insufficiency.

5. 14-Hydroxy-4β,5-methano-3β-[α-L-rhamnopyranosyl)oxy]-5β,14β-bufa-20,22-dienolide.

6. 14 - Hydroxy - 4β,5 - methano - 3β - [(2,3 - O - isopropylidene - α - L - rhamnopyranosyl)-oxy] - 5β,14β - bufa - 20,22, dienolide.

7. 14 - Hydroxy - 4β,5 - methano - 3β - [(2,3 - O - isopropylidene - 4 - O - methyl - α - L - rhamno-pyranosyl)oxy]-5β,14β-bufa-20,22-dienolide.

8. 14-Hydroxy-4β,5-methano-3β-[(2,3-O-ethoxymethylidene-α-L-rhamnopyranosyl)oxy]-5β,14β-bufa-20,22-dienolide.

9. 14-Hydroxy-4β,5-methano-3β-[(4-O-methyl-α-L-rhamnopyranosyl)oxy]-5β,14β-bufa-20,22-dienolide.

**Patentanspruch für den Vertragsstaat: Österreich (AT)**

Verfahren zur Herstellung von 4,5-Methano-bufadienolid-rhamnosiden der Formel I

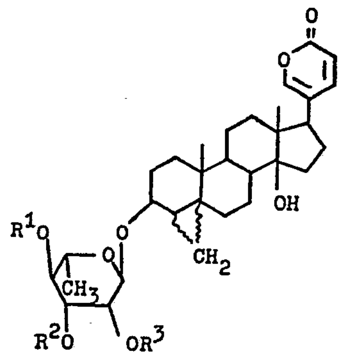

I,

worin $R^1$ ein Wasserstoffatom oder einen Alkyl- oder Acylrest mit jeweils 1 bis 4 Kohlenstoffatomen bedeutet,

$R^2$ und $R^3$ gleich oder verschieden sind und Wasserstoffatome, Alkyl- oder Acylreste mit jeweils 1 bis 4 Kohlenstoffatomen oder beide zusammen den Rest

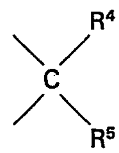

darstellen, worin $R^4$ und $R^5$ gleich oder verschieden sind und Wasserstoffatome oder Alkyl- oder Alkoxygruppen mit jeweils 1 bis 2 Kohlenstoffatomen darstellen, dadurch gekennzeichnet, daß man

a) ein 3,14-Dihydroxy-4,5-methano-14β-bufa-20,22-dienolid der allgemeinen Formel II

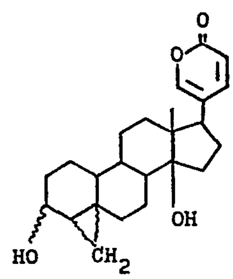

II

mit einem Rhamnosederivat der allgemeinen Formel III

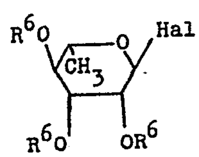

III

worin $R^6$ Acetylgruppen oder Benzoylgruppen darstellen und Hal ein Halogenatom bedeutet, umsetzt und anschließend gegebenenfalls die so erhaltenen Verbindungen in an sich bekannter Weise verseift und danach verethert und/oder in sie den Rest

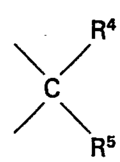

einführt, worin $R^4$ und $R^5$ daselbe wie oben bedeuten, oder

b) — falls die Verbindung der allgemeinen Formel I ein cis-Methano-proscillaridinderivat darstellt — an eine Verbindung der allgemeinen Formel IV

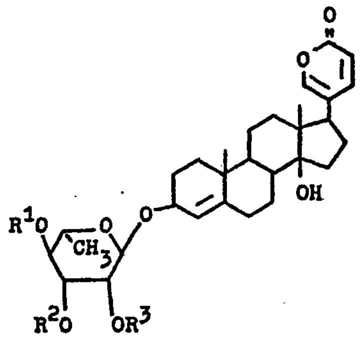

IV

worin $R^1$, $R^2$ und $R^3$ dasselbe wie oben bedeuten, Methylen addiert.

**Revendication pour l'Etat contractant: AT**

1. Procédé de préparation de 4,5-méthano-bufadienoliderhamnoside de formule I

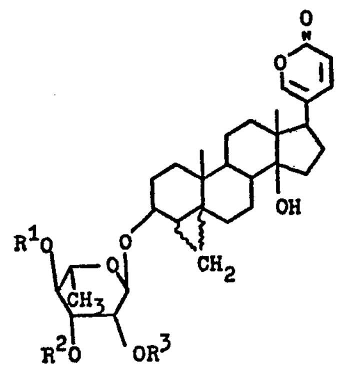

dans laquelle $R^1$ représente un atome d'hydrogène ou un reste alkyle ou acyle ayant 1 à 4 atomes de carbone, $R^2$ et $R^3$ sont identiques ou différents et représentent des atomes d'hydrogène, des restes alkyl ou acyle ayant 1 à 4 atomes de carbone ou bien les deux ensemble, le reste

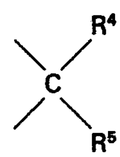

où $R^4$ et $R^5$ sont identiques ou différents et représentent des atomes d'hydrogène ou des groupes alkyle ou alcoxy ayant 1 à 2 atomes de carbone, caractérisé par le fait que

a) on fait réagir un 3,14-dihydroxy-4,5-méthano-14$\beta$-bufa-20,22-diénolide de formule générale II

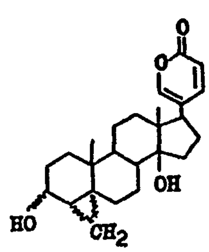

avec un dérivé de rhamnoside de formule générale III

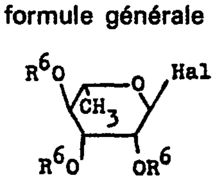

dans laquelle $R^6$ représente des groupes acétyle ou benzoyle et Hal un atome d'halogène puis, éventuellement, on saponifie de manière connue les composés ainsi obtenus et enfin on estérifie et/ou on y introduit le reste

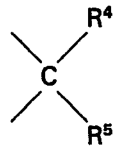

, dans lequel $R^4$ et $R^5$ ont la même signification que ci-dessus, ou

b) dans le cas où le composé de formule générale I représente un dérivé de cis-méthano-proscillaridine — on additionne de méthylène un composé de formule générale IV

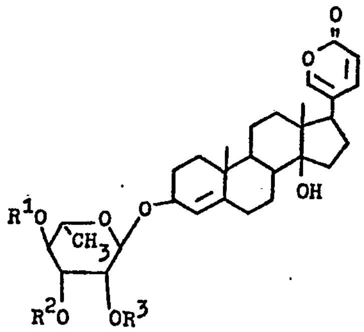

dans laquelle $R^1$, $R^2$ et $R^3$ ont la même signification que ci-dessus.

**Claim for the Contracting State: AT**

A process for the preparation of a 4,5-methano-bufadienoliderhamnoside of the formula I

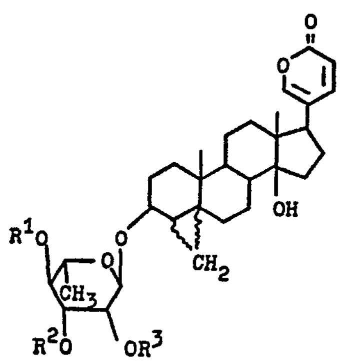

I

where $R^1$ is hydrogen or alkyl of 1 to 4 carbon atoms or acyl of 1 to 4 carbon atoms, and $R^2$ and $R^3$ are identical or different and each is hydrogen or alkyl of 1 to 4 carbon atoms or acyl of 1 to 4 carbon atoms, or both together are

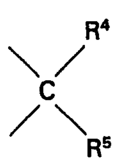

where $R^4$ and $R^5$ are identical or different and each is hydrogen or alkyl or alkoxy of 1 or 2 carbon atoms, wherein

a) a 3,14-dihydroxy-4,5-methano-14β-bufa-20,22-dienolide of the general formula II

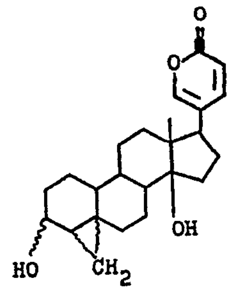

II

is reacted with a rhamnose derivative of the general formula III

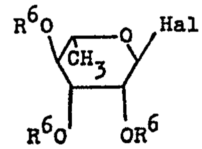

III

where the $R^6$ groups are acetyl or benzoyl and Hal is halogen, and if desired the resulting compound is hydrolyzed in a conventional manner and then if desired etherified and/or if desired a radical

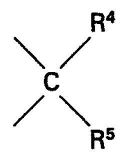

where $R^4$ and $R^5$ have the above meanings, is introduced into the resulting compound, or

b) if the compound of the general formula I to be produced is a cis-methano-proscillaridine derivative, a methylene group is introduced into a compound of the general formula IV

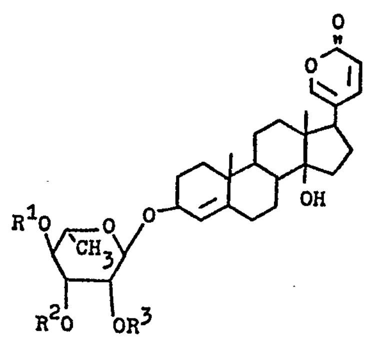

IV

where $R^1$, $R^2$ and $R^3$ have the same meanings as above.